# EUROPEAN PATENT APPLICATION

(11) **EP 1 806 605 A1**
(43) Date of publication of application: **11.07.2007**
(21) Application number: 05765593.8
(22) Date of filing: 11.07.2005
(51) Int. Cl.: G02B 5/22, G02B 5/28, C09B 23/00, C09B 69/02, C09K 3/00, C07D 405/14, C07D 209/14, C07D 215/06, C07D 277/64, C07D 417/08, C07D 401/14, C07D 209/60

(54) **FILTER AND CYANINE COMPOUND**

(30) Priority: 12.07.2004 JP 2004204982
(71) Applicant: NIPPON KAYAKU KABUSHIKI KAISHA, Tokyo 102-8172 (JP)
(72) Inventor: TORINIWA, Toshitaka NIPPON KAYAKU KK, 3-26-8, Shimo Kita-ku, Tokyo (JP); KONDO, Terumasa, Kitaadumi-gun, Nagano 3998602 (JP); IKEDA, Masaaki NIPPON KAYAKU KK, 3-26-8, Shimo, Kitaku, Tokyo (JP)
(74) Representative: Gille Hrabal Struck Neidlein Prop Roos
(86) International application number: PCT/JP2005/012791
(87) International publication number: WO 2006/006573

(57) **Abstract**

An optical filter, which is capable of cutting-off unnecessary near infrared rays, neon light or the like, and preventing reflection of fluorescent light or the like, in plasma display panels and the like, at the same time, superior in transmittance for visible light, and also superior in heat resistance, moisture resistance, and the like of the coloring matter contained, had been desired. The present invention was made to solve such problems. The present invention relates to an optical filter comprising a salt of a cation of a cyanine coloring matter with a di(halogenoalkylsulfonyl)imide anion, preferably a cyanine compound represented by the following formula: 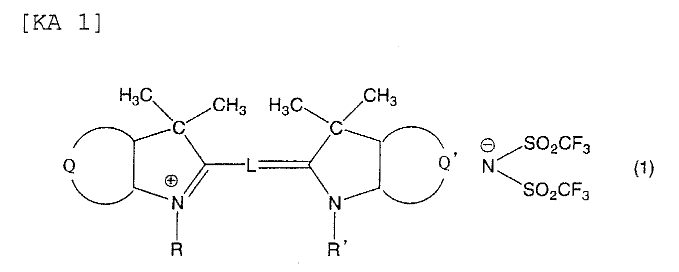 (wherein, in the above formula (1), each of Q and Q' independently represents a benzene ring or a naphthalene ring which may have a substituent; each of R and R' independently represents an alkyl group or an alkoxyalkyl group; and L represents a linking group to form a carbocyanine), and useful as a filter for cutting-off near infrared rays, improving image characteristics for displays or the like.

## Description

### Technical Field

The present invention relates to an optical filter and a cyanine compound. More specifically, the present invention relates to an optical filter comprising a cyanine compound and absorbing red to near infrared rays, a material which can be used for said filter comprising said compound and absorbing red to near infrared rays, and a novel cyanine compound. Further more specifically, the present invention relates to an optical filter comprising a cyanine compound having a high molecular absorbance coefficient (mass absorbance coefficient) and a very sharp absorption waveform, and is suitable for cutting-off near infrared rays and/or improving image characteristics particularly for displays such as plasma display TV, a resin composition comprising said cyanine compound, and a novel cyanine compound.

### Background Art

Recently, a large-sized flat-screen TV which has been intensively studied and a plasma display panel (hereinafter, abbreviated as PDP) which has been attracting attentions as a display need an electromagnetic interference shielding material, a near infrared rays cutting-off filter, a neon light cutting-off filter, or the like, to shield electromagnetic waves, near infrared rays, neon light, and the like, which are inevitably generated due to the mechanisms thereof. And many studies have been done for these materials. For example, near infrared rays cutting-off filter has been disclosed in Patent Literature 1, and the like. At the same time, coloring matters for shielding emission lines originated from neon and the like, and/or preventing reflection of fluorescent light, and improving image characteristics, and molded resin products such as film or optical filter using said coloring matter or the like are studied. As the coloring matter for improving image characteristics, a coloring matter having a sharp absorption at a specified wavelength is needed in order to shield unnecessary near infrared rays, neon light, and the like, which are inevitably generated. By using an optical filter containing such a coloring matter for improving image characteristics, image characteristics of an image display device can be significantly improved. However, in view of heat resistance, light resistance, absorption characteristics for unnecessary near infrared rays and neon light, transmittance for visible light, and the like of a coloring matter to be used, a satisfactory coloring matter has not been provided.
Conventionally, as a red to infrared rays absorbing material, several cyanine compounds had been known (see, for example, Patent Literatures 2 and 3). Among these compounds, compounds whose counter ion is antimony hexafluoride ion had been mainly used due to superior heat resistance thereof. However, since antimony-containing compounds correspond to deleterious substances, a compound not containing a heavy metal had been desired in recent years, in the industry fields where use of heavy metals or the like is regulated, in particular, in the electric materials field.

Patent Literature 1: JP-A-2000-81511
Patent Literature 2: JP-B-5-37119
Patent Literature 3: JP No. 3045404

### Disclosure of Invention

### Problem to be Solved by the Invention

The present invention was made under the consideration of such circumstances, and an object of the present invention is to provide an optical filter which can improve image characteristics by cutting off infrared rays, neon light and the like impeditive to image characteristics or the like, and having superior transmittance for visible light, as well as maintain said performances for a long period of time, and also a novel cyanine compound suitable to the filter, which is superior in moisture resistance, heat resistance, and light resistance, in particular, in wet heat resistance.

### Means for Solving the Problem

The present inventors have, after intensively studying a way to solve the above problem, completed the present invention. Thus, the present invention relates to:
(1) An optical filter comprising a salt of a cation of a cyanine coloring matter with a di(halogenoalkylsulfonyl)imide anion.
(2) The optical filter according to the above item (1), wherein the salt of a cation of a cyanine coloring matter with a di(halogenoalkylsulfonyl)imide anion is represented by the following formula (1) : 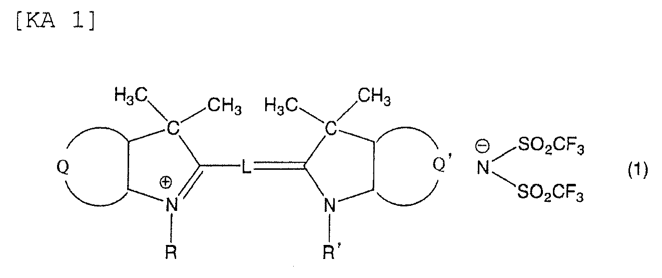 (wherein, in the above formula (1), each of Q and Q' independently represents a benzene ring or a naphthalene ring which may have a substituent; each of R and R' independently represents an alkyl group or an alkoxyalkyl group; and L represents a linking group to form a carbocyanine).
(3) The optical filter according to the above item (2), wherein L in the formula (1) is one group selected from the group consisting of carbocyanine crosslinking groups represented by the following formulae: 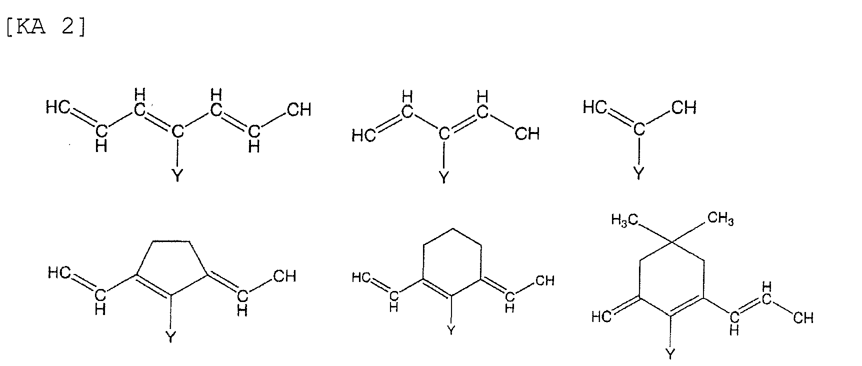 (wherein Y represents a halogen atom, a diphenylamino group, or a lower alkyl group).
(4) The optical filter according to the above item (2) or (3), wherein R and R' in the formula (1) are a methyl group or a methoxyethyl group.
(5) The optical filter according to any one of the above items (1) to (4), wherein said filter is for cutting-off near infrared rays, improving image characteristics, or both thereof.
(6) The optical filter according to any one of the above items (1) to (5), wherein said filter is for a plasma display panel.
(7) The optical filter according to any one of the above items (1) to (6), further comprising, as a near infrared rays absorbing agent, any coloring matter of a diimonium compound, a phthalocyanine type compound, or a nickel complex type compound.
(8) The optical filter according to the above item (7), wherein an anion of diimonium compound is the same anion as a cyanine coloring matter.
(9) A cyanine compound represented by the following formula (1) : 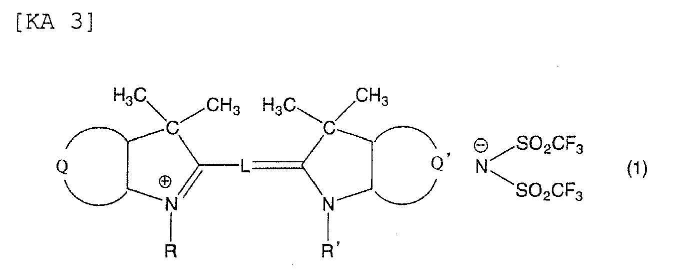 {wherein, in the above formula (1), each of Q and Q' independently represents a benzene ring which may be substituted by a halogen atom or methoxy, or a naphthalene ring which may be substituted by a halogen atom or methoxy; each of R and R' independently represents an alkyl group or an alkoxyalkyl group; and L represents any one of the linking groups represented by the following formulae: 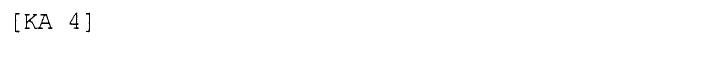 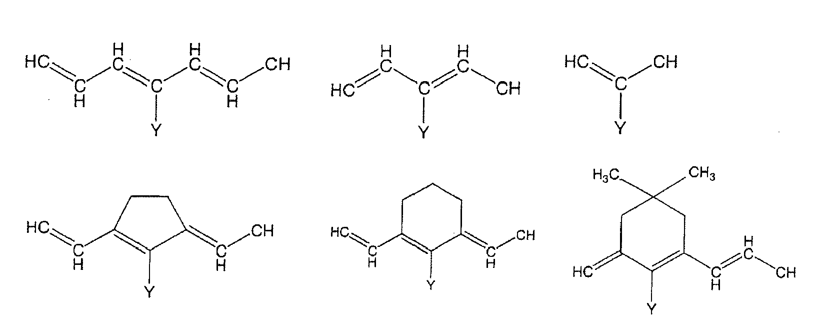 (wherein Y represents a halogen atom, a diphenylamino group, or a lower alkyl group)}.
(10) The cyanine compound according to the above item (9), wherein, in the formula (1), an indole group having Q or Q' linking to L is a group represented by an indole group represented by the following formula (4) (in the case of an indole group having Q', the formula (4) should be read as a corresponding structural formula): 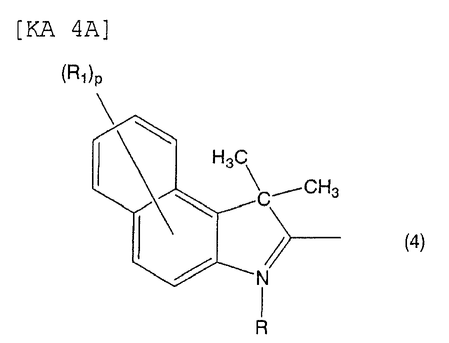 (wherein R₁ represents a halogen atom, an alkyl group, or an alkoxyalkyl group; p represents 0 or an integer not larger than 4; and R means the same as in the above formula (1)).
(11) The cyanine compound according to the above item (9) or (10), wherein, in the formula (1), the linking group represented by L is a linking group represented by the following formula (9): 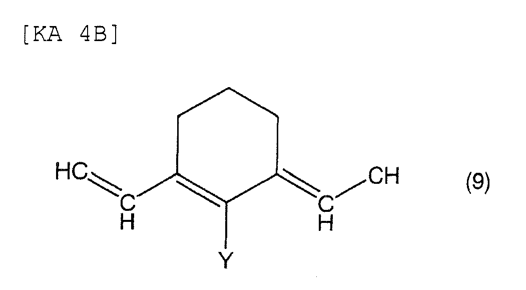 (wherein Y represents a halogen atom, a diphenylamino group, or a lower alkyl group).
(12) The cyanine compound according to the above item (9) or (10), wherein, in the formula (1), the linking group represented by L is a linking group represented by the following formula (8): 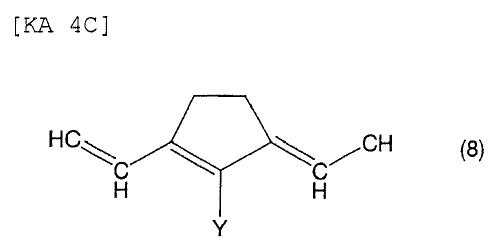 (wherein Y represents a halogen atom, a diphenylamino group, or a lower alkyl group).

### Effect of the Invention

The salt of a cyanine coloring matter with a di(halogenoalkylsulfonyl)imide to be used in the present invention is a compound, which does not contain antimony, arsenic or the like nor correspond to deleterious substances, and has a high molar absorbance coefficient selectively in the wavelength regions including infrared region and other regions relating to emission lines originating from neon and the like and/or reflection of fluorescent light, and is also superior in heat resistance, light resistance, solubility, and the like. Further, compared with the conventional salt with hexafluorophosphate, said compound is more superior, in particular, in heat resistance. Therefore, the optical filter of the present invention characterized by comprising said compound does not contain antimony or the like, and is extremely superior in heat resistance and hardly gives rise to reactions such as decomposition due to heat. Due to such features, the optical filter of the present invention is suitable to, for example, a near infrared rays absorbing filter for plasma display and an optical filter for improving image characteristics. Further, the optical filter can be also used for applications such as heat insulating films and sunglasses.

### Best Mode for Carrying Out the Invention

Hereinafter, the present invention will be explained in detail. The optical filter of the present invention comprises a salt of a cyanine coloring matter with a di (halogenoalkylsulfonyl) imide (a compound comprising a cation of a cyanine coloring matter and a di(halogenoalkylsulfonyl)imide anion). As the di(halogenoalkylsulfonyl)imide anion in said salt, di(trifluoromethylsulfonyl)imide anion is preferable. Compounds having di(trifluoromethylsulfonyl)imide anion preferably include a compound represented by the following formula (1) :

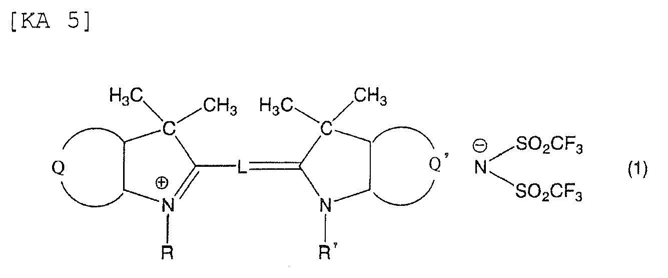

(wherein each of Q and Q' independently represents a benzene ring or a naphthalene ring which may have a substituent; each of R and R' independently represents an alkyl group or an alkoxyalkyl group; and L represents a linking group to form a carbocyanine). Here, the substituent, which the benzene ring or the naphthalene ring of Q and Q' may have, includes a halogen atom, an alkyl group and an alkoxyalkyl group, preferably a halogen atom and a methoxy group.

In the above formula (1), specific examples of the indole group having Q and Q' include groups represented by the following formulae (2) to (4). However, in the case of Q' , the indole group in the following formulae should be read as structures corresponding to the indole group having Q' in the formula (1).

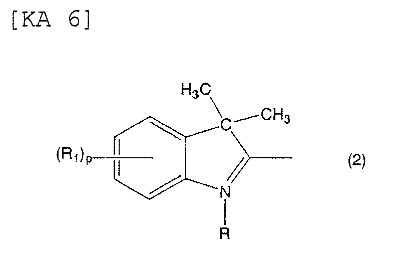

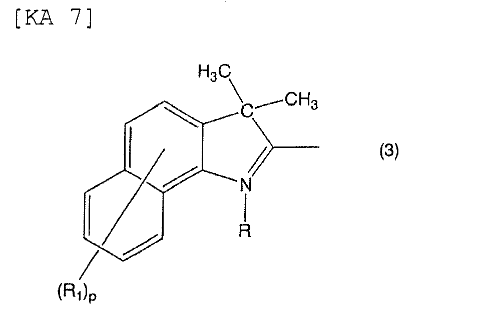

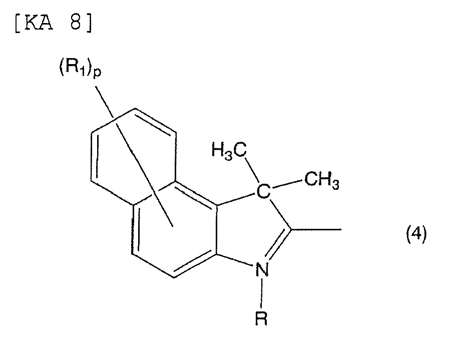

In the above formulae (2) to (4), R₁ represents a halogen atom, an alkyl group or an alkoxyalkyl group; and p represents 0 or an integer not larger than 4. Further, R means the same as in the above formula (1). In the above formula (1), L represents a linking group to formmono-, di- or tri-carbocyanine, and is preferably any one of the following formulae (5) to (10).

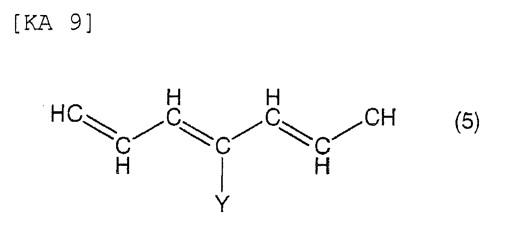

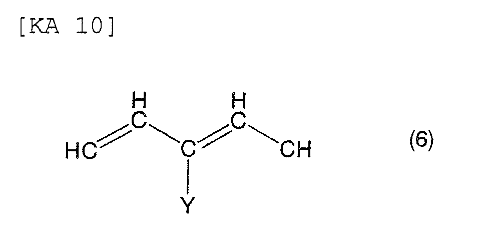

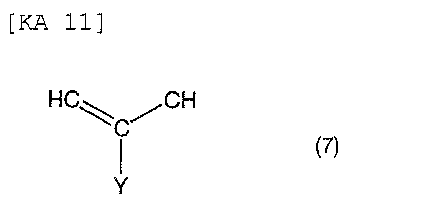

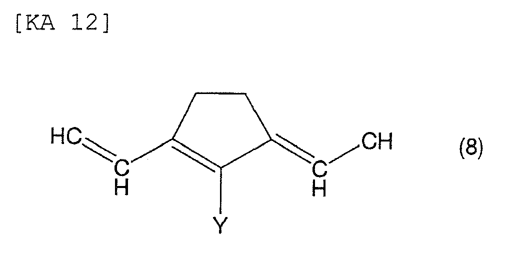

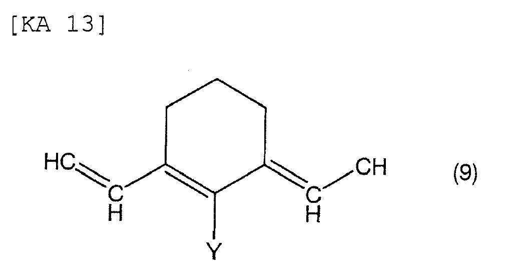

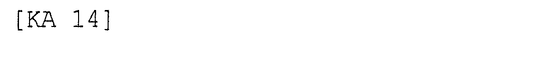

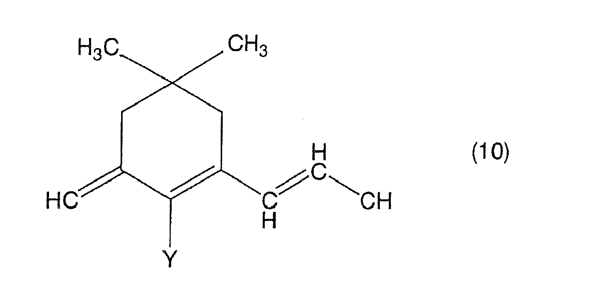

In the formulae (5) to (10), Y represents a hydrogen atom; a halogen atom such as fluorine atom, chlorine atom, bromine atom, and the like; a diphenylamino group; or a C1-C4 lower alkyl group such as a methyl group, an ethyl group, an i-propyl group, a n-butyl group, an i-butyl group, a t-butyl group, and the like; and is preferably a chlorine atom.

In the formula (1), each of R and R' independently represents an alkyl group or an alkoxyalkyl group. The alkyl group includes, for example, a C1-C5 alkyl group such as a methyl group, an ethyl group, a n-propyl group, an i-propyl group, a n-butyl group, an i-butyl group, a t-butyl group, a n-amyl group, an i-amyl group and a t-amyl group, and the alkoxyalkyl group includes, for example, a (C1-C4)alkoxy(C1-C4)alkyl group such as a methoxymethyl group, a methoxyethyl group or an ethoxyethyl group. R or R' is preferably a methyl group or a methoxyethyl group, and preferably both of R and R' are the same groups to each other from the viewpoint of easiness in synthesis.

The alkyl group in the di(halogenoalkylsulfonyl)imide anion includes a saturated or an unsaturated, straight, branched or cyclic alkyl group generally having 1 to 36 carbon atoms. The alkyl group is preferably a saturated straight alkyl group having 1 to 20 carbon atoms, preferably 1 to 10 carbon atoms, further preferably 1 to 4 carbon atoms, and most preferably 1 carbon atom, which may have a substituent. The halogen atom in the di(halogenoalkylsulfonyl)imide anion is preferably a fluorine atom, a chlorine atom, a bromine atom or an iodine atom, more preferably a fluorine atom , a chlorine atom or a bromine atom, and most preferably a fluorine atom. The halogenoalkyl group in the di(halogenoalkylsulfonyl)imide anion includes a trifluoromethyl group, a difluoromethyl group, a monofluoromethyl group, a dichloromethyl group, a monochloromethyl group, a dibromomethyl group, a difluorochloromethyl group, a pentafluoroethyl group, a tetrafluoroethyl group, a trifluoroethyl group, a trifluorochloroethyl group, a difluoroethyl group, a monofluoroethyl group, a trifluoroiodoethyl group, a heptafluoropropyl group, a hexafluoropropyl group, a pentafluoropropyl group, a tetrafluoropropyl group, a trifluoropropyl group, a difluoropropyl group, a monofluoropropyl group, a perfluorobutyl group, a perfluorohexyl group, a perfluorooctyl group, a perfluorooctylethyl group, a pentafluoroisopropyl group, a heptafluoroisopropyl group, a perfluoro-3-methylbutyl group and a perfluoro-3-methylhexyl group, and is preferably a trifluoromethyl group.

The cyanine compound represented by the formula (1) can be produced through various processes, for example, through the following processes described in Patent Literature 3. Namely, 2 moles of a compound represented by the formula (11) :

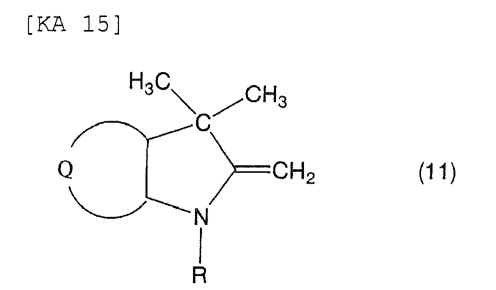

(wherein, in the formula (11), Q and R mean the same as in the above formula (1)), 1 mole of a compound represented by the formula (12) or the formula (13):

[KA 16]

OHC-L=CHOH (12)

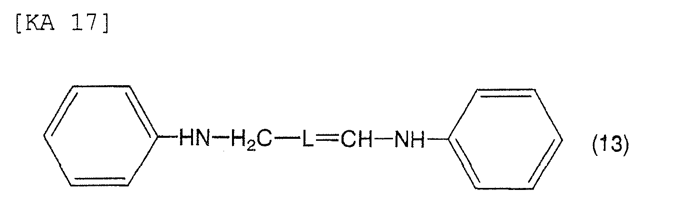

(wherein, in the formula (12) and the formula (13), L means the same as in the above formula (1));

and 1 mole of a salt such as a salt of the di(halogenoalkylsulfonyl)imide with an alkali metal are condensed, if necessary, in the presence of a basic catalyst such as sodium acetate, potassium acetate, piperazine and piperidine, in a dehydrating organic acid such as acetic anhydride or a mixture of acetic anhydride and glacial acetic acid, under the heating conditions, for example, at 50 to 140°C for generally 10 to 12 hours, preferably 10 to 60 minutes, to synthesize a symmetrical cyanine compound (a compound where Q' and R' in the formula (1) are same to Q and R, respectively). An asymmetrical cyanine compound can be synthesized by the same procedure as in the above description except that 1 mole of a compound of the formula (11) and 1 mole of another compound of the formula (11) which has different Q and R from the above compound (Q' and R' different from Q and R in the formula (1)) are used instead of 2 moles of a compound of the formula (11).

Alternatively, 1 mole of a compound of the formula (14):

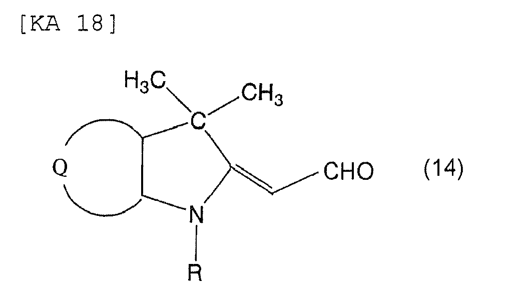

(wherein Q and R mean the same as in the formula (1)), 1 mole of a compound of the formula (15):

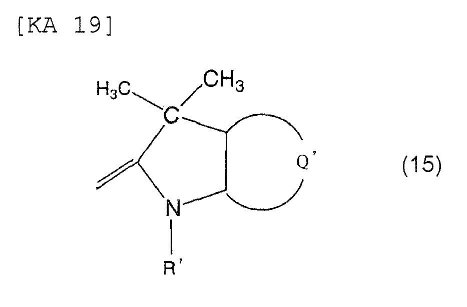

(wherein Q' and R' mean the same as in the formula (1)), and 1 mole of a salt of the di(halogenoalkylsulfonyl)imide are condensed, if necessary, in the presence of a basic catalyst such as sodium acetate, potassium acetate, piperazine and piperidine, in a dehydrating organic acid such as acetic anhydride or a mixture of acetic anhydride and glacial acetic acid, under the heating conditions, for example, at 50 to 140°C for generally 10 to 12 hours, preferably 10 to 60 minutes, to synthesize a symmetrical or an asymmetrical cyanine compound. The product may be purified by recrystallizing from methanol, ethanol, or other organic solvent, if necessary.

Next, specific examples of the salt of a cation of the cyanine coloring matter with a di(halogenoalkylsulfonyl)imide anion to be used in the present invention are shown below. However, examples of the compounds to be used in the present invention are not limited to these compounds.

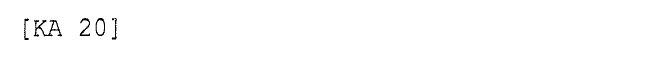

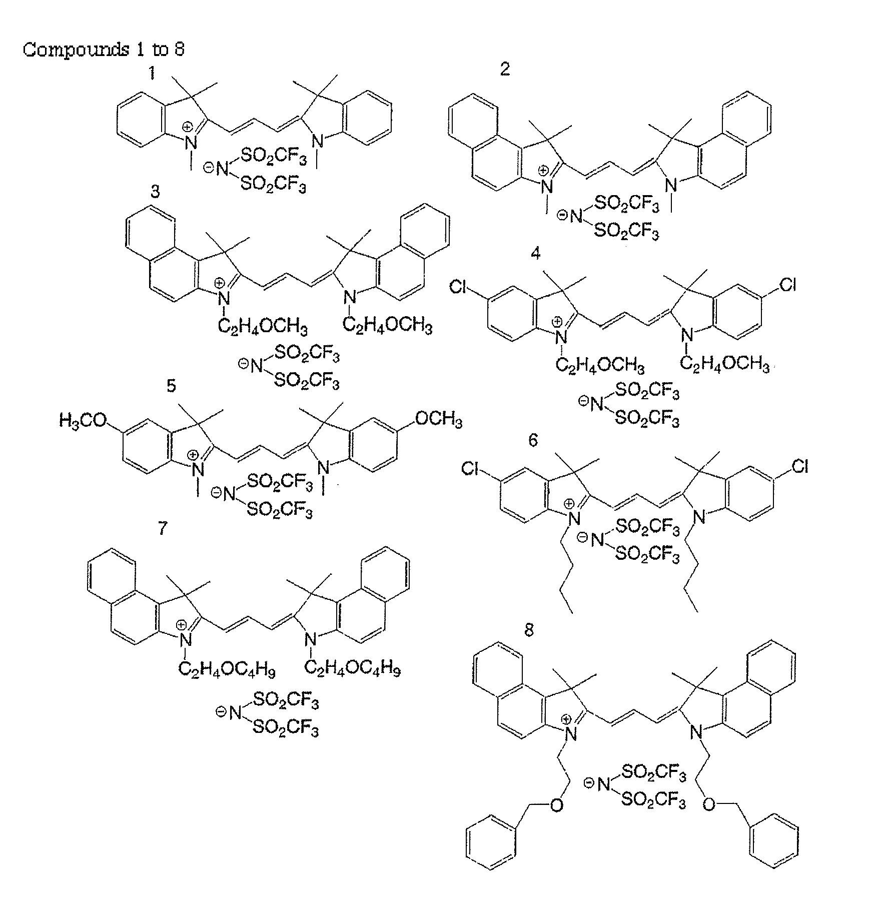

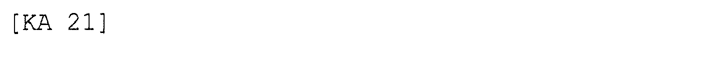

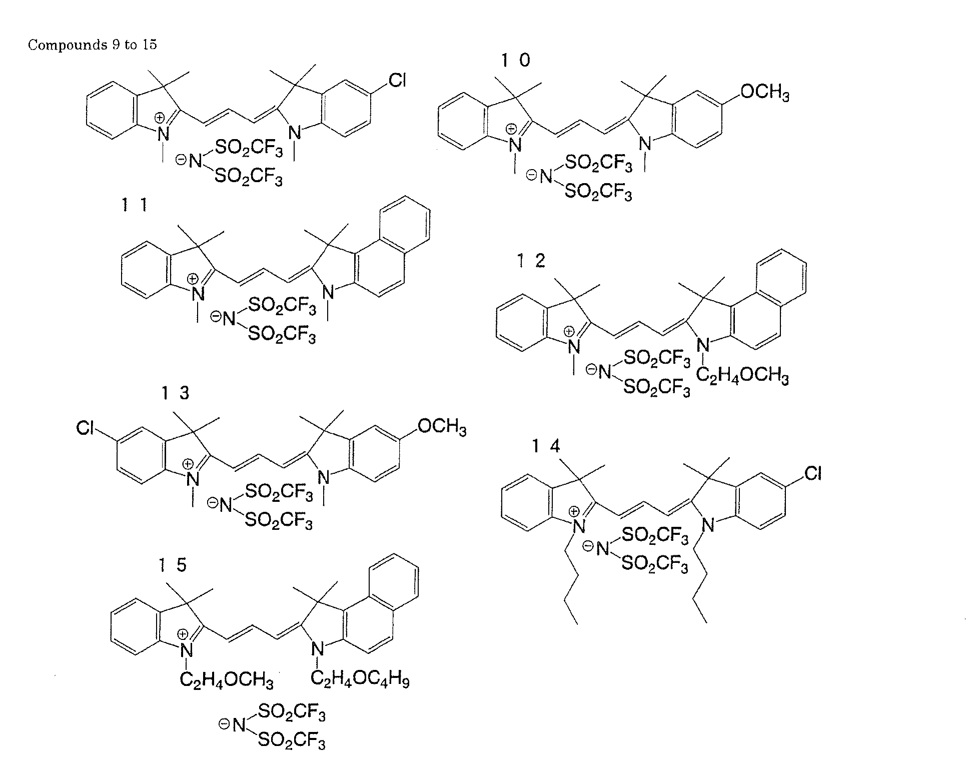

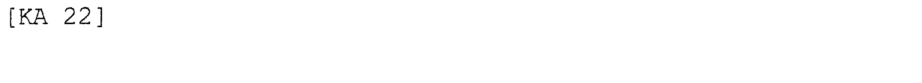

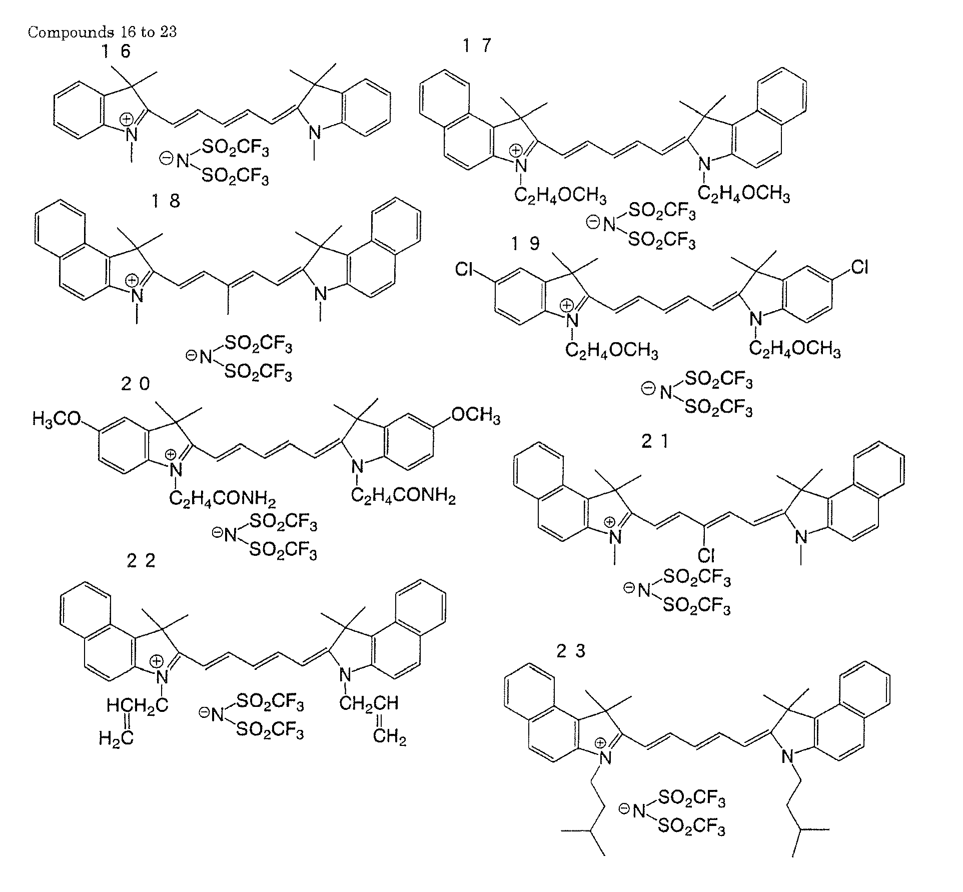

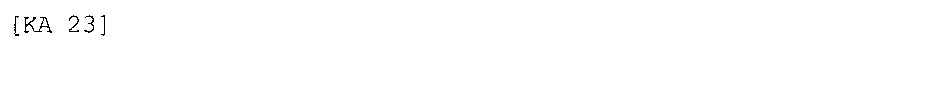

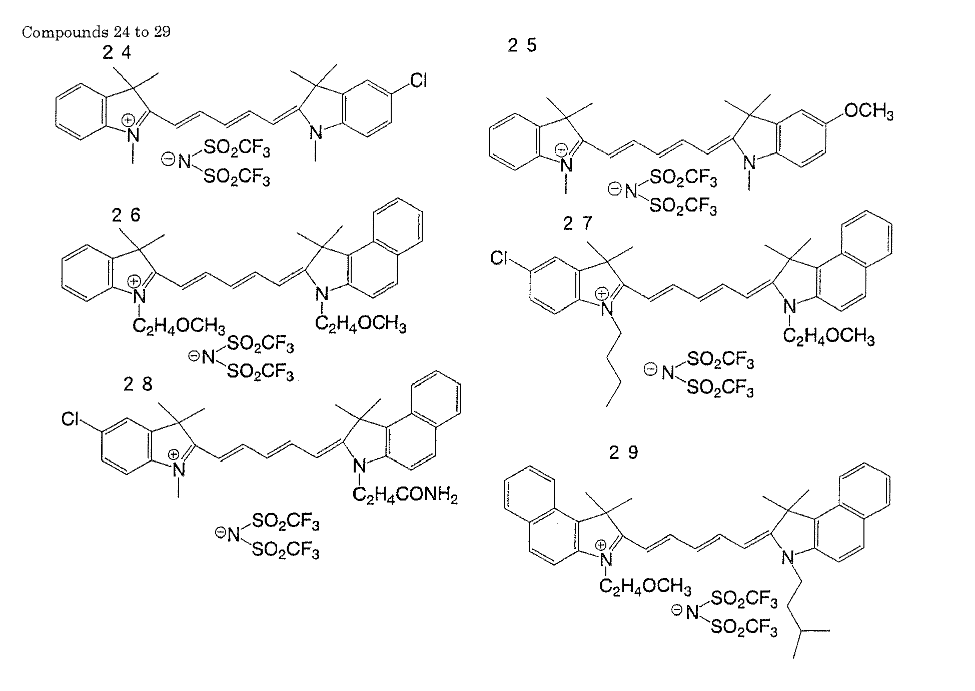

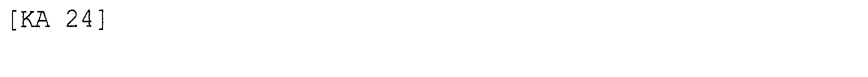

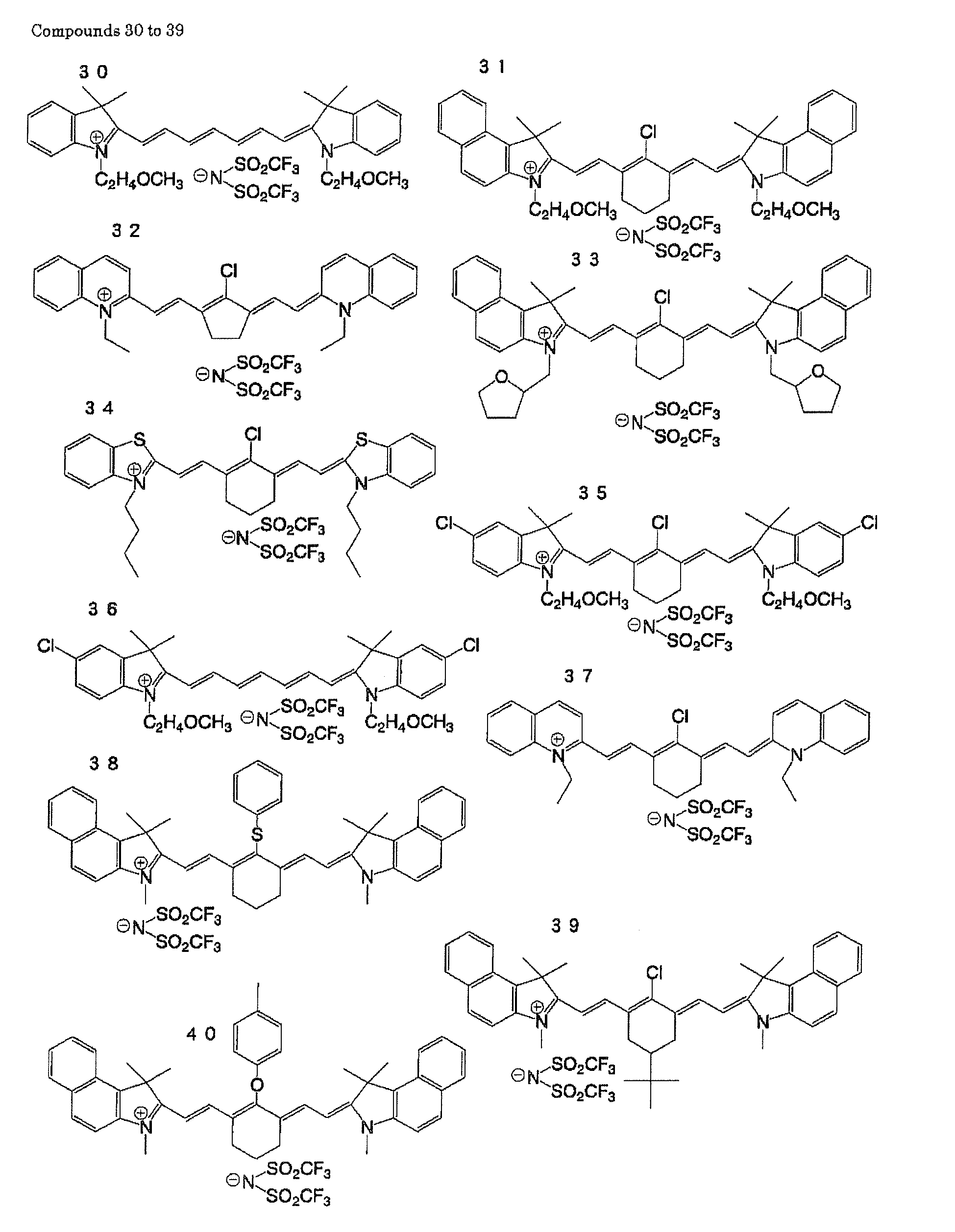

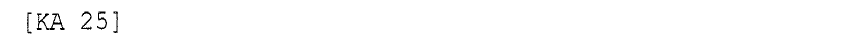

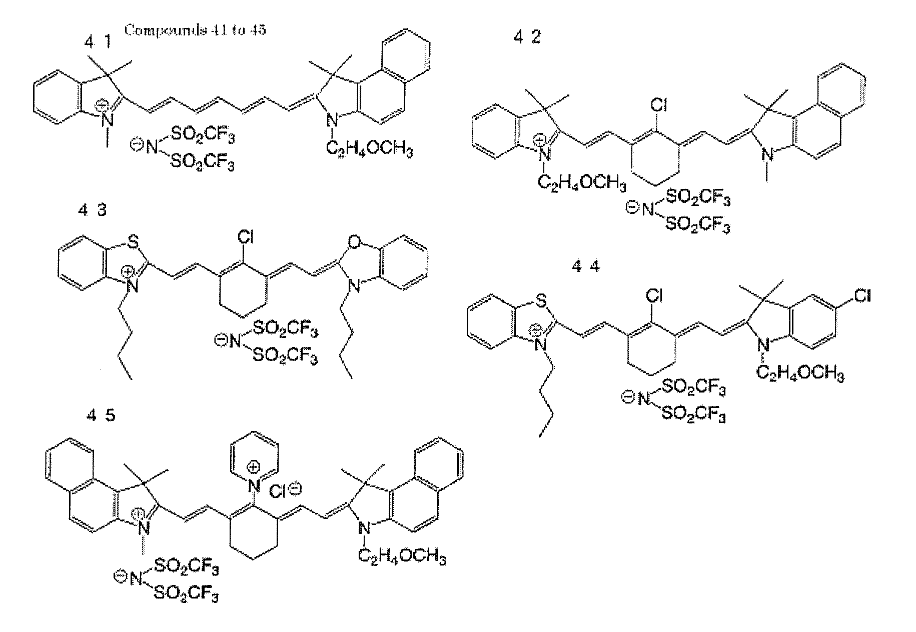

The cyanine compound to be used in the present invention, preferably the cyanine compound of the present invention, is suitably used as a near infrared rays absorbing coloring matter for a near infrared rays absorbing filter, a near infrared rays absorbing coloring matter for an optical filter, a coloring matter for improving image characteristics to be used for obtaining neutral gray and the like. The cyanine compound can be also used in an optical information recording medium.

The optical filter of the present invention can be obtained typically by dissolving the cyanine compound to be used in the present invention in a solvent or the like, mixing, with a resin liquid or the like, if necessary, and then coating or molding. In the optical filter of the present invention, a way by which the cyanine compound is contained, is not particularly limited, so long as the compound is contained so that unnecessary near infrared rays can be cut off. For example, a solution containing said cyanine compound may be coated on a substrate, or may be coated on a substrate together with a binder resin to form a film. Or otherwise, a resin layer containing said cyanine compound may be provided on a substrate. Alternatively, a substrate itself may be a layer comprising a resin composition (or hardened body thereof) containing the cyanine compound to be used in the present invention. Generally, substrate is not particularly limited, so long as the substrate can be used for the optical filter or the like, but usually a substrate made of a resin is used. Thickness of the layer is usually around 0.1 µm to 10 mm, but it is decided, as appropriate, depending on, the target of cutoff rate for near infrared rays, or the like. Further, content of the cyanine compound to be used in the present invention is also decided, as appropriate, depending on the target of cutoff rate for near infrared rays or the like. Usually, concentration of said cyanine compound and thickness of the layer are preferably adjusted so that a cutoff rate for near infrared rays or the like as a target becomes not less than 50%, preferably not less than 60% with a film layer containing said cyanine compound having a thickness of 0.5 to 10 µm. Concentration of said cyanine compound to be compounded in the resin varies depending on absorbance of the compound, film thickness, target cutoff rate, and the like, therefore it is not categorically described, but it is usually around 0.01 to 40 parts in a film layer containing said cyanine compound, and around 0.1 to 30 parts in the case of a film layer having the above thickness based on 100 parts of resin. The resin to be used is not particularly limited so long as the resin is capable of forming a film layer, and includes vinyl compounds such as polyethylene, polystyrene, poly(acrylic acid), poly(acrylate ester), poly(vinyl acetate), polyacrylonitrile, poly(vinyl chloride), poly(vinyl fluoride) and addition polymers of these vinyl compounds; poly(methacrylic acid); poly(methacrylate ester); poly(vinylidene chloride); poly (vinylidene fluoride); poly(vinylidene cyanide); copolymers of a vinyl compound or a fluorine type compound such as vinylidene fluoride / trifluoroethylene copolymer, vinylidene fluoride / tetrafluoroethylene copolymer, and vinylidene cyanide / vinyl acetate copolymer; fluorine-containing resins such as poly(trifluoroethylene), poly(tetrafluoroethylene) and poly(hexafluoropropylene); polyamides such as nylon 6 and nylon 66; polyimides; polyurethanes; polypeptides; polyesters such as polyethylene terephthalate; polycarbonates; polyethers such as polyoxymethylene; epoxy resins; poly(vinyl alcohol); poly(vinyl butyral); and the like.

Method for preparing the optical filter of the present invention is not particularly limited, and, for example, the following methods well-known per se can be utilized.
(1) A method to produce a resin plate or a film by kneading the cyanine compound to be used in the present invention with a resin followed by hot forming;
(2) A method to produce a resin plate or a film by performing cast polymerization the cyanine compound to be used in the present invention with a resin monomer or a prepolymer of a resin monomer in the presence of a polymerization catalyst;
(3) A method by preparing a coating composition containing the cyanine compound to be used in the present invention and coating on a transparent resin plate, a transparent film, or a transparent glass plate;
(4) A method to produce a laminated resin plate, a laminated resin film, or a laminated glass plate by using a composition containing the cyanine compound to be used in the present invention and a resin (adhesive).

The production method of (1) generally includes a method to produce a resin plate by adding the cyanine coloring matter to be used in the present invention to powder or pellet of a substrate resin and heating to 150 to 350°C to dissolve followed by forming, a method to make a film (resin plate) using an extruder and the like, although processing temperature and film(plate)-making conditions, etc. differ to some extent depending on the resin to be used. Amount of the cyanine coloring matter of the present invention to be added varies depending on thickness, absorption intensity, transmittance for visible light, and the like of a resin plate or a film to be made, but it is usually 0.01 to 30% by mass, preferably 0.03 to 15% by mass based on the mass of substrate resin.

In the method (2), forming is performed by injecting the above compound and a resin monomer or a prepolymer of a resin monomer into a mold in the presence of a polymerization catalyst to react to harden therein, or casting into a metal mold to solidify to a hard product in the mold. A number of resins can be formed by this process, and specific examples of the resin, to which such a method can be applied, include acrylic resin, diethyleneglycol-bis(allylcarbonate) resin, epoxy resin, phenol-formaldehyde resin, polystyrene resin, silicone resin, and the like. Among them, casting method by mass polymerization of methyl methacrylate which gives an acrylic resin sheet superior in hardness, heat resistance and chemical resistance, is preferable.

As polymerization catalyst, well-known radical thermal polymerization initiators can be utilized. Those initiators include peroxides such as benzoyl peroxide, p-chlorobenzoyl peroxide, diisopropyl peroxicarbonate, and the like; and azo compounds such as azobisisobutyronitrile. Amount thereof to be used is generally 0.01 to 5% by mass based on the total amount of the mixture. Heating temperature in the thermal polymerization is generally 40 to 200°C, and polymerization time is generally around 30 minutes to 8 hours. Further, besides the thermal polymerization, photopolymerization method can be employed by adding a photopolymerization initiator or a sensitizer.

The method (3) includes a method to prepare a coating composition by dissolving the cyanine coloring matter to be used in the present invention in a binder resin and an organic solvent, a method to prepare an aqueous coating composition by pulverizing and dispersing the above compound in the presence of a resin, and the like. Among these, in the method to prepare a coating composition by dissolving the cyanine coloring matter to be used in the present invention in a binder resin and an organic solvent, the resin which can be used as a binder include, for example, aliphatic ester resin, acryl-based resin, melamine resin, urethane resin, aromatic ester resin, polycarbonate resin, polyvinyl-based resin, aliphatic polyolefin resin, aromatic polyolefin resin, polyvinyl alcohol resin, modified polyvinyl resin, and the like, or copolymer resin thereof.

As a solvent, a solvent of halogen type, alcohol type, ketone type, ester type, aliphatic hydrocarbon type, aromatic hydrocarbon type and ether type, or a mixture thereof can be used. Concentration of the cyanine coloring matter to be used in the present invention varies depending on thickness, absorption intensity and transmittance for visible light of a coating layer to be prepared, but is generally 0.1 to 30% by mass, based on the binder resin.

Using the thus produced coating composition, coating can be performed on a transparent resin film, a transparent resin plate, a transparent glass plate, or the like using a spin coater, a bar coater, a roll coater, spraying, or the like to obtain a near infrared rays absorbing filter.
In the method (4), as a resin for adhesive, well-known transparent adhesives for resin such as silicone type, urethane type, acryl type or the like, or for laminated glass such as polyvinyl butyral adhesive for laminated glass, ethylene / vinyl acetate type adhesive or the like, can be used. Using an adhesive containing 0.1 to 30% by mass of the cyanine coloring matter to be used in the present invention, transparent resin plates with each other, a resin plate with a resin film, a resin plate with a glass, resin films with each other, a resin film with a glass, and glass plates with each other are adhered to produce an optical filter.
In this connection, when kneading or mixing is performed in each method, an additive usually used in resin forming such as UV absorber, plasticizer, or the like may be added.

The optical filter, in particular, a filter for absorbing near infrared rays of the present invention may be produced by comprising one or more kinds of the cyanine compounds to be used in the present invention only as a near infrared rays absorbing compound, or further comprising a near infrared rays absorbing compound other than the cyanine compound to be used in the present invention in combination. Other near infrared rays absorbing compound which can be used in combination includes, for example, diimonium type coloring matter, phthalocyanine type coloring matter, metal complex coloring matter such as nickel dithiol complex. When basic structure of these other near infrared rays absorbing compounds capable of being used in combination is a cationic coloring matter, a counter anion thereof is preferably the same anion to the cyanine compound to be used in the present invention. In particular, the near infrared rays absorbing compound is preferably a diimonium type coloring matter, and further preferably a diimonium type coloring matter whose anion is the same anion to the cyanine coloring matter to be used in the present invention. Further, examples of inorganic metal near infrared rays absorbing compound capable of being used in combination include, for example, copper compound such as metal copper, copper sulfide and copper oxide, metal mixture containing zinc oxide as a main component, tungsten compound, ITO, ATO, and the like.

Further, even when the optical filter of the present invention is an optical filter for improving image characteristics (mainly for cutting-off neon light other than near infrared rays and/or for preventing reflection of fluorescent light), the optical filter may comprise one or more kinds of the cyanine compounds of the present invention only which has absorbability in said wavelength region, or further comprise the above near infrared rays absorbing compound in combination. When a near infrared rays absorbing compound is used in combination, an optical filter having both functions of absorption of near infrared rays and improvement of image characteristics can be obtained.
Still further, in order to change color tone of the optical filter, the cyanine compound to be used in the present invention or other coloring matter may be added as a coloring matter having an absorption in the visible region (a coloring matter for improving image characteristics). Alternatively, an optical filter containing a coloring matter for improving image characteristics only is produced, and thereafter the optical filter can be laminated with the optical filter for absorbing near infrared rays of the present invention.

In such an optical filter for absorbing near infrared rays and the like, when it is used as a front plate of a plasma display, transmittance for visible light is desirably as high as possible, and needs to be not less than 40%, preferably not less than 50%.
The near infrared rays region to be cut-off is preferably 750 to 1,200 nm, more preferably 800 to 1,000 nm, and a desirable average transmittance of near infrared rays in this region is not more than 50%, more preferably not more than 30%, further more preferably not more than 20%, and particularly preferably not more than 10%.
Further, as a coloring matter for improving image characteristics, because unnecessary neon light other than the ideal emissions of three primary colors is absorbed, preferably a coloring matter having a sharp absorption at a specified wavelength region of around 580 to 620 nm is used. Furthermore, a superior optical filter can be obtained by using, for example, a coloring matter having a sharp absorption in a region of 490 to 550 nm as a countermeasure for reflection of fluorescent light, and a coloring matter having a sharp absorption in a region of 560 to 580 nm to absorb an emission of xenon, respectively. Thus, in the optical filter of the present invention, the cyanine compound may be selected so that the coloring matter has a sharp absorption at any region in the wavelengths of 490 to 1,200 nm corresponding to the purpose.
As the cyanine compound to be used in the present invention, a compound, whose maximum absorption wavelength is located within the wavelength region to be cut-off, is preferably used.

The filter of the present invention for absorbing near infrared rays can be used not only for an application such as a front plate of displays, but for a filter or a film which needs to cut-off near infrared rays, such as heat insulating film, optical product, sunglasses, and the like.
The optical filter of the present invention does not contain antimony nor arsenic, and is environment-friendly and more superior in stability compared with a conventional near infrared rays absorbing filter which does not contain antimony but contains perchlorate ion, hexafluorophosphate ion or fluoroborate ion. Furthermore, the filter of the present invention has a sufficient solubility and is superior in processability. In particular, since the filter of the present invention is extremely superior in heat resistance, wet heat resistance and light resistance, and hardly brings on a reaction such as heat decomposition, an optical filter which hardly gives rise to coloring in the visible region can be obtained. Further, due to such features, the filter is suitable to a near infrared rays absorbing filter for plasma display or the like. Still further, the filter can be suitably used for a near infrared rays absorbing film such as heat insulating film and sunglasses, or the like.

Hereinafter, the present invention will be further specifically explained using Examples. In Examples, parts and % mean parts by mass and % by mass, respectively, unless otherwise noted.
In this connection, absorbance (OD value) was measured using a spectrophotometer UV-3150 made by Shimadzu Corp. When measurement was carried out using a solvent, the same solvent was measured as a blank, and when measurement was carried out using a filter, a filter which had the same composition except that only the cyanine compound was not contained was produced and measured as a blank.

### Example 1

### Synthesis of Compound 1

A mixture of 9.6 parts of 1,3,3-trimethyl-2-methyleneindoline, 10.6 parts of 1,3,3-trimethyl-2-formylmethyleneindoline, 17.6 parts of potassium (bis-trifluoromethanesulfonyl)imide represented by the following formula:

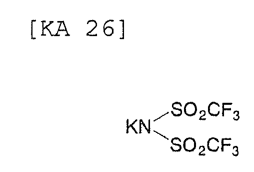

in 75 parts of acetic anhydride was boiled under cooling-reflux for 1 hour, then cooled to room temperature. Subsequently, the reaction liquid was filtered under aspiration to remove insoluble impurity. Thereafter, 100 parts of water was added dropwise to the reaction liquid. The precipitated crystal was filtered under aspiration, recrystallized from 40 parts of methanol, washed with 5 parts of methanol, then with water, and dried, to obtain 19.4 parts of the aforementioned Compound 1. Spectroscopic characteristics of the resultant Compound 1 were as follows.
Maximum absorption wavelength: 544 nm (in methanol);
Molar absorbance coefficient: 133,000 (in methanol).

### Example 2

### Synthesis of Compound 3

Into a mixed solvents of 50 parts of acetic anhydride and 25 parts of acetic acid, 14.9 parts of 4,5-benzo-1-(2-methoxyethyl)-3,3-dimethyl-2-methyleneindoli ne, 8.9 parts of triethyl orthoformate, 8.8 parts of potassium (bis-trifluoromethanesulfonyl)imide and 2.5 parts of concentrated hydrochloric acid were charged, and the reaction mixture was boiled under cooling-reflux for 2 hours, then cooled to room temperature. Subsequently, 25 parts of water was added, and the precipitated crystal was filtered under aspiration. The crystal was boiled in 15 parts of isopropyl alcohol under cooling-reflux for 1 hour, and then cooled to room temperature. The crystal was filtered under aspiration, washed with 5 parts of isopropyl alcohol, then with water, and dried, to obtain 13. 4 parts of the aforementioned Compound 3. Spectroscopic characteristics of the resultant Compound 3 were as follows.
Maximum absorption wavelength: 588 nm (in methanol);
Molar absorbance coefficient: 119,000 (in methanol).

### Example 3

### Synthesis of Compound 12

The same procedures were repeated as in Example 1 except that 14.9 parts of 4,5-benzo-1-(2-methoxyethyl)-3,3-dimethyl-2-methyleneindoline was used instead of 9.6 parts of 1,3,3-trimethyl-2-methyleneindoline, to obtain 18.3 parts of the aforementioned Compound 12. Spectroscopic characteristics of the resultant Compound 12 were as follows.
Maximum absorption wavelength: 555 nm (in methanol);
Molar absorbance coefficient: 119,000 (in methanol).

### Example 4

### Synthesis of Compound 17

Into 50 parts of acetic acid, 14.9 parts of 4,5-benzo-1-(2-methoxyethyl)-3,3-dimethyl-2-methyleneindoli ne, 6.5 parts of malonaldehydedianyl·hydrochloride, and 4.1 parts of sodium acetate were charged and the reaction mixture was heated up to 60°C. 5.1 parts of acetic anhydride was added dropwise thereto at 50°C to 60°C over 1 hour, and then stirred at 50°C to 60°C for further 2 hours to complete the condensation reaction. The reaction liquid was cooled to room temperature, and then filtered under aspiration to remove insoluble impurity. Subsequently, a solution of 8.8 parts of potassium (bis-trifluoromethanesulfonyl)imide in 40 parts of water was added dropwise to the reaction liquid. The precipitated crystal was filtered under aspiration, recrystallized from 40 parts of methanol, washed with 5 parts of methanol, then with water, and dried, to obtain 14.9 parts of the aforementioned Compound 17. Spectroscopic characteristics of the resultant Compound 17 were as follows.
Maximum absorption wavelength: 680 nm (in methanol);
Molar absorbance coefficient: 220,000 (in methanol).

### Example 5

### Synthesis of Compound 31

Into 25 parts of acetic acid, 14.9 parts of 4,5-benzo-1-(2-methoxyethyl)-3,3-dimethyl-2-methyleneindoli ne and 4.5 parts of 2-chloro-1-formyl-3-hydroxymethylenecyclohexene were charged and the reaction mixture was heated up to 60°C. 5.1 parts of acetic anhydride was added dropwise thereto at 50°C to 60°C over 1 hour, and then stirred at 50°C to 60°C for further 2 hours to complete the condensation reaction. The reaction liquid was cooled to room temperature, and then filtered under aspiration to remove insoluble impurity. Subsequently, a solution of 8.8 parts of potassium (bis-trifluoromethanesulfonyl)imide in 40 parts of water was added dropwise to the reaction liquid. The precipitated crystal was filtered under aspiration, recrystallized from 70 parts of N,N-dimethylformamide, washed with 5 parts of methanol, then with water, and dried, to obtain 19.1 parts of the aforementioned Compound 31. Spectroscopic characteristics and decomposition temperature of the resultant Compound 31 were as follows. In this connection, decomposition temperature was shown by a value of the temperature at which weight loss initiated in a thermogravimetric - differential thermal analysis (TG-DTA).
Maximum absorption wavelength: 820 nm (in methanol);
Molar absorbance coefficient: 270,000 (in methanol);
Decomposition temperature: 242.9°C (TG-DTA).
Further, solubility to various types of solvents at room temperature was as follows.
Dichloromethane: 19.2%;
N,N-dimethylformamide: 24.6%;
Methyl ethyl ketone(MEK): 2.9%;
Cyclopentanone: 14.5%.

### Example 6

### Synthesis of Compound 35

Into 25 parts of acetic acid, 13.2 parts of 5-chloro-1-(2-methoxyethyl)-3,3-dimethyl-2-methyleneindolin e and 3.6 parts of 2-chloro-1-formyl-3-hydroxymethylenecyclohexene were charged and the reaction mixture was heated up to 60°C. 4.1 parts of acetic anhydride was added dropwise thereto at 50°C to 60°C over 2 hours, then stirred at 50°C to 60°C for further 1 hour to complete the condensation reaction. The reaction liquid was cooled to room temperature, and then filtered under aspiration to remove insoluble impurity. Subsequently, a solution of 7.0 parts of potassium (bis-trifluoromethanesulfonyl)imide in 45 parts of water was added dropwise to the reaction liquid. The precipitated crystal was filtered under aspiration, recrystallized from 15 parts of N,N-dimethylformamide, washed with 5 parts of methanol, then with water, and dried, to obtain 10.2 parts of the aforementioned Compound 35. Spectroscopic characteristics and decomposition temperature of the resultant Compound 35 were as follows.
Maximum absorption wavelength: 790 nm (in methanol);
Molar absorbance coefficient: 285,000 (in methanol);
Decomposition temperature: 226.3°C (TG-DTA).

### Example 7

### Synthesis of Compound 36

The same procedures were repeated as in Example 6 except that 5.7 parts of glutaconic acid aldehydedianil·hydrochloride was used instead of 3.6 parts of 2-chloro-1-formyl-3-hydroxymethylenecyclohexene, to obtain 9.9 parts of the aforementioned Compound 36. Spectroscopic characteristics and decomposition temperature of the resultant Compound 36 were as follows.
Maximum absorption wavelength: 751 nm (in methanol);
Molar absorbance coefficient: 273,000 (in methanol);
Decomposition temperature: 226.6°C (TG-DTA).

### Example 8

### Synthesis of Compound A

The same procedures were repeated as in Example 6 except that 10.2 parts of 1-(2-methoxyethyl)-3,3-dimethyl-2-methyleneindoline was used instead of 13.2 parts of 5-chloro-1-(2-methoxyethyl)-3,3-dimethyl-2-methyleneindolin e, to obtain 8.8 parts of the following Compound A. Spectroscopic characteristics and decomposition temperature of the resultant Compound A below were as follows.
Maximum absorption wavelength: 780 nm (in methanol);
Molar absorbance coefficient: 278,000 (in methanol);
Decomposition temperature: 224.6°C (TG-DTA).

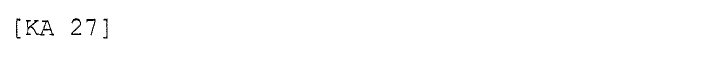

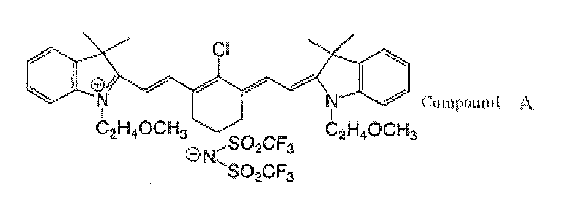

### Example 9

### Synthesis of Compound 33

The same procedures were repeated as in Example 6 except that 15.3 parts of 3,3-dimethyl-2-methylene-1-(tetrahydrofurfuryl)methylindoli ne represented by the following formula:

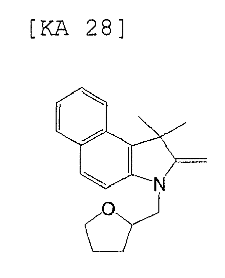

was used instead of 13.2 parts of 5-chloro-1-(2-methoxyethyl)-3,3-dimethyl-2-methyleneindolin e, to obtain 16.4 parts of the aforementioned Compound 33. Spectroscopic characteristics of the resultant Compound 33 were as follows.
Maximum absorption wavelength: 823 nm (in methanol);
Molar absorbance coefficient: 264,000 (in methanol).

### Example 10

### Synthesis of Compound 34

Into 50 parts of ethanol, 17.5 parts of 1-n-butyl-2-methylbenzothiazolium iodide and 3.6 parts of 2-chloro-1-formyl-3-hydroxymethylenecyclohexene were charged and the reaction mixture was heated up to 60°C. 10.6 parts of triethylamine was added dropwise thereto at 50°C to 60°C over 1 hour, then stirred at 50°C to 60°C for further 1 hour to complete the condensation reaction. The reaction liquid was cooled to room temperature, and then filtered under aspiration to remove insoluble impurity. Subsequently, a solution of 7.0 parts of potassium (bis-trifluoromethanesulfonyl)imide in 45 parts of water was added dropwise to the reaction liquid. The precipitated crystal was filtered under aspiration, recrystallized from 25 parts of N,N-dimethylformamide, washed with 15 parts of methanol, then with water, and dried, to obtain 3.3 parts of the aforementioned Compound 34. Spectroscopic characteristics of the resultant Compound 34 were as follows.
Maximum absorption wavelength: 797 nm (in methanol);
Molar absorbance coefficient: 331,000 (in methanol).

### Example 11

### Synthesis of Compound 37

Into 50 parts of ethanol, 15.7 parts of 1-ethyl-2-methylquinolinium iodide, 3.6 parts of 2-chloro-1-formyl-3- hydroxymethylenecyclohexene and 7.0 parts of potassium (bis-trifluoromethanesulfonyl)imide were charged and the reaction mixture was heated up to 60°C. 10.6 parts of triethylamine was added dropwise thereto at 50°C to 60°C over 1 hour, then stirred at 50°C to 60°C for further 1 hour to complete the condensation reaction. The reaction liquid was cooled to room temperature. The precipitated crystal was filtered under aspiration, and then boiled in 50 parts of methanol under cooling-reflux for 1 hour. Subsequently, the reaction liquid was cooled to room temperature. The precipitated crystal was filtered under aspiration, washed with 15 parts of methanol, then with water, and dried, to obtain 11.4 parts of the aforementioned Compound 37. Spectroscopic characteristics of the resultant Compound 37 were as follows.
Maximum absorption wavelength: 846 nm (in methanol);
Molar absorbance coefficient: 235,000 (in methanol).

### Example 12

### Filter containing Compound 31 and heat resistance stability test thereof

In 18.8 parts of MEK, 0.4 parts of Compound 31 obtained in the above Example 5 was dissolved. To this solution, 80 parts of a resin solution prepared by dissolving 25 parts of an acryl-based resin (DIANAL (RTM) BR-80, made by Mitsubishi Rayon Co., Ltd.) in 75 parts of MEK was mixed to obtain a solution for coating. This solution was coated on a polyester film so that thickness becomes 2 to 4 µm, dried at 80°C to obtain an optical filter of the present invention. This filter was used as a filter for absorbing near infrared rays.
The resultant filter for absorbing near infrared rays was left to stand in an oven at 80°C for 7 days. Then absorbance (OD value) of the filter was measured with a spectrophotometer before and after the test, and residual ratio of the coloring matter was determined from the change of the absorbance (OD value) at the maximum absorption wavelength. The results of the heat resistance test are shown in Table 1.
Residual ratio = (Absorbance after the test / Absorbance before the test) x 100

### Example 13

### Filter containing Compound 35 and heat resistance stability test thereof

The same procedures were repeated as in Example 12 using Compound 35 obtained in the above Example 6, to produce a filter, which was evaluated similarly. The results are shown in Table 1.

### Example 14

### Filters containing Compounds 30, A, B and C, respectively, and heat resistance stability tests thereof

The same procedures were repeated as in Example 12 using the above Compound 30, Compound A obtained in the above Example, the following Compound B and Compound C, respectively, to produce filters containing each of above compounds, and evaluation thereof was carried out in the same manner as in Example 12. The results are shown in Table 2.

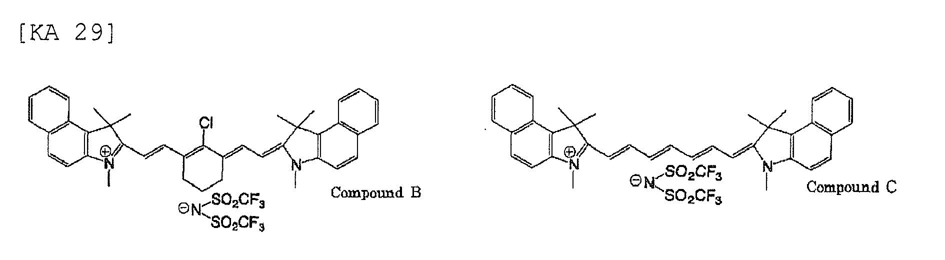

### Comparative Example 1

The same procedures were repeated as in Example 5 except that sodium hexafluoroantimonate was used instead of potassium (bis-trifluoromethanesulfonyl)imide, to synthesize a hexafluoroantimonate salt (SbF₆ salt), and the same procedures were repeated as in Example 12 except that this compound was used, to produce a filter, which was evaluated similarly. The results are shown in Table 1.

### Comparative Example 2

A compound in which the (bis-trifluoromethanesulfonyl)imide salt in Compound 35 obtained in Example 6 was replaced with hexafluoroantimonate salt (SbF₆ salt) was synthesized in the same manner as in Comparative Example 1, then a filter was produced using the compound in the same manner as in Example 13, and evaluated similarly. The results are shown in Table 1.

### Comparative Examples 3 to 6

The same procedures were repeated as in Example 5 except that sodium tetrafluoroborate and sodium p-toluenesulfonate was used instead of potassium (bis-trifluoromethanesulfonyl)imide, to synthesize tetrafluoroborate salt (BF₄ salt) (Comparative Example 3) and p-toluenesulfonate salt (PTS salt) (Comparative Example 4), respectively, instead of the (bis-trifluoromethanesulfonyl)imide salt in Compound 31. Then the same procedures were repeated as in Example 12 except that each of these compounds was used, to produce filters, which were evaluated similarly. The results are shown in Table 1.
Similarly to the above, compounds in which the (bis-trifluoromethanesulfonyl)imide salt in Compound 35 obtained in Example 6 was replaced with a tetrafluoroborate salt (BF₄ salt) (Comparative Example 5) and a p-toluenesulfonate salt (PTS salt) (Comparative Example 6), respectively, were synthesized. Then the same procedures were repeated as in Example 13 except that each of these compounds was used, to produce filters, which were evaluated similarly. The results are shown in Table 1.

[Table 1]

**Table 1, Heat resistance stability test**

| Compound No. Compound No. | Absorbance (OD value) | | Residual Ratio |
|---|---|---|---|
| | Initial | After 7 days | |
| Example 12 (Compound 31) | 0.65 | 0.52 | 80.9% |
| Comparative Example 1 (SbF₆ salt) | 0.66 | 0.50 | 75.0% |
| Comparative Example 3 (BF₄ salt) | 0.71 | 0.55 | 77.4% |
| Comparative Example 4 (PTS salt) | 0.51 | 0.36 | 69.5% |
| Example 13 (Compound 35) | 0.76 | 0.67 | 89.1% |
| Comparative Example 2 (SbF₆ salt) | 0.73 | 0.58 | 79.2% |
| Comparative Example 5 (BF₄ salt) | 0.77 | 0.62 | 81.2% |
| Comparative Example 6 (PTS salt) | 0.55 | 0.35 | 62.8% |

[Table 2]

**Table 2, Heat resistance stability test**

| Compound No. | Absorbance (OD value) | | Residual Ratio |
|---|---|---|---|
| | Initial | After 7 days | |
| Example 14 (Compound 30) | 0.83 | 0.74 | 89.7% |
| Example 14 (Compound A) | 0.83 | 0.72 | 87.2% |
| Example 14 (Compound B) | 0.67 | 0.57 | 85.6% |
| Example 14 (Compound C) | 0.75 | 0.61 | 80.9% |

From Table 1, it is understood that the filters produced using the compounds of the present invention ((bis-trifluoromethanesulfonyl)imide salts) are superior in stability under high temperature condition from their higher residual ratios compared with those of the filters produced using the compounds of Comparative Examples having different kinds of anions.
From Table 2, it is understood that the other compounds of the present invention are also superior in stability under high temperature condition.

### Example 15

### Wet heat resistance stability test of the filter in Example 12

The filter obtained in Example 12 (the filter using Compound 31) was left to stand in a high temperature constant humidity chamber at 85°C and 85% RH for 3 days. Absorbance (OD value) of the filter was measured with a spectrophotometer before and after the test, and residual ratio of the coloring matter was determined from the change of the absorbance (OD value) at the maximum absorption wavelength.

### Example 16

### Wet heat resistance stability test of the filter in Example 13 (the filter using Compound 35)

The same procedures were repeated as in Example 12 except that the filter obtained in Example 13 was used, to perform the wet heat resistance test, and residual ratio of the coloring matter was determined. The results are shown in Table 3.

### Example 17

### Wet heat resistance stability test of the filter in Example 14

The same procedures were repeated as in Example 12 except that the filters obtained in Example 14 (the filters using each of Compounds 30, A, B and C, respectively) were used, to perform the wet heat resistance test, and residual ratios of the coloring matters were determined. The results are shown in Table 3.

### Comparative Examples 7 to 12

The filters obtained in Comparative Examples 1 to 6 were left to stand in a high temperature constant humidity chamber at 85°C and 85% RH for 3 days. Then absorbances (OD values) of the filters were measured with a spectrophotometer before and after the test, and residual ratios of the coloring matters were determined from the changes of the absorbances (OD values) at each maximum absorption wavelength. The test using the filter obtained in Comparative Example 1 is termed as Comparative Example 7, the test using the filter obtained in Comparative Example 2 is termed as Comparative Example 8, the test using the filter obtained in Comparative Example 3 is termed as Comparative Example 9, the test using the filter obtained in Comparative Example 4 is termed as Comparative Example 10, the test using the filter obtained in Comparative Example 5 is termed as Comparative Example 11, and the test using the filter obtained in Comparative Example 6 is termed as Comparative Example 12. The results are shown in Table 3.

[Table 3]

**Table 3, Wet heat resistance stability test**

| Compound No. | Absorbance (OD value) | | Residual Ratio |
|---|---|---|---|
| | Initial | After 3 days | |
| Example 15 (Compound 31) | 0.65 | 0.59 | 90.9% |
| Comparative Example 7 (SbF₆ salt) | 0.66 | 0.54 | 81.0% |
| Comparative Example 9 (BF₄ salt) | 0.71 | 0.63 | 88.2% |
| Comparative Example 10 (PTS salt) | 0.51 | 0.43 | 84.2% |
| Example 16 (Compound 35) | 0.76 | 0.71 | 93.9% |
| Comparative Example 8 (SbF₆ salt) | 0.73 | 0.62 | 84.9% |
| Comparative Example 11 (BF₄ salt) | 0.77 | 0.61 | 79.0% |
| Comparative Example 12 (PTS salt) | 0.55 | 0.46 | 83.2% |
| Example 17 (Compound 30) | 0.83 | 0.80 | 97.3% |
| Example 17 (Compound A) | 0.83 | 0.82 | 99.9% |
| Example 17 (Compound B) | 0.67 | 0.63 | 93.6% |
| Example 17 (Compound C) | 0.75 | 0.67 | 89.7% |

From Table 3, it is understood that the filters produced using the compounds obtained in Examples of the present invention are also superior in high temperature and high humidity condition from higher residual ratios of coloring matters compared with those of the filters produced using the compounds obtained in Comparative Examples.

### Example 18

The same procedures were repeated as in Example 5 except that 9.1 parts of (2-chloro-3-phenylaminomethylene-cyclopenta-1-enylmethylene )-phenylammonium·hydrochloride represented by the following formula:

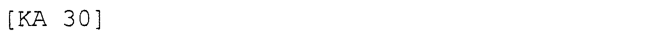

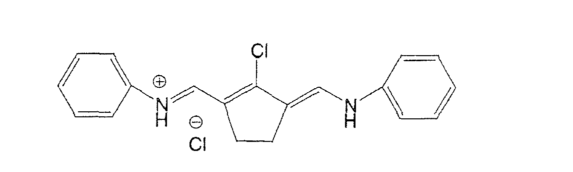

was used instead of 4.5 parts of 2-chloro-1-formyl-3-hydroxymethylenecyclohexene, to obtain 17 parts of Compound 46 represented by the following formula:

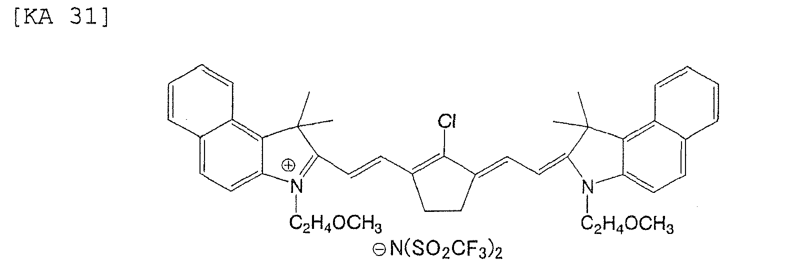

Spectroscopic characteristics and decomposition temperature of the resultant Compound 46 were as follows. In this connection, decomposition temperature was shown by a value of the temperature at which weight loss initiated in a thermogravimetric - differential thermal analysis (TG-DTA).
Maximum absorption wavelength: 845 nm (in methanol);
Molar absorbance coefficient: 275,000 (in methanol);
Decomposition temperature: 213.0°C (TG-DTA).
Further, solubility to various kinds of solvents at room temperature was as follows.
N,N-dimethylformamide: 3.4%;
Methyl ethyl ketone (MEK): 0.2%;
Cyclopentanone: 3.1%;
Cyclohexanone: 0.8%.

### Example 19

The same procedures were repeated as in Example 5 except that 17.3 parts of 4,5-benzo-1-(2-n-butoxyethyl)-3,3-dimethyl-2-methyleneindol ine was used instead of 14.9 parts of 4,5-benzo-1-(2-methoxyethyl)-3,3-dimethyl-2-methyleneindoli ne, to obtain 18.6 parts of Compound 47 represented by the following formula:

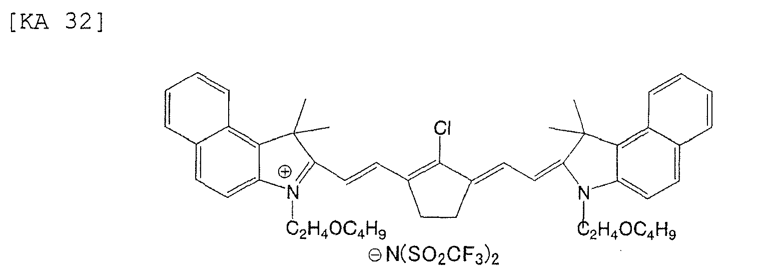

Spectroscopic characteristics and decomposition temperature of the resultant Compound 47 were as follows. In this connection, decomposition temperature was shown by a value of the temperature at which weight loss initiated in a thermogravimetric - differential thermal analysis (TG-DTA).
Maximum absorption wavelength: 845 nm (in methanol);
Molar absorbance coefficient: 275,000 (in methanol);
Decomposition temperature: 186.0°C (TG-DTA).
Further, solubility to various kinds of solvents at room temperature was as follows.
N,N-dimethylformamide: 4.5%;
Methyl ethyl ketone (MEK): 1.6%;
Cyclopentanone: 0.9%;
Cyclohexanone: 2.6%.

### Industrial Applicability

Since the optical filter of the present invention is capable of, for example, cutting-off near infrared rays, neon light or the like, as well as superior in transmittance for visible light, and at the same time, the coloring matter contained in said filter is also superior in wet heat resistance and the like, the optical filter of the present invention is useful as an optical filter for plasma displays, etc., and the like.

## Claims

1. An optical filter comprising a salt of a cation of a cyanine coloring matter with a di (halogenoalkylsulfonyl) imide anion.

2. The optical filter according to claim 1, wherein the salt of a cation of a cyanine coloring matter with a di(halogenoalkylsulfonyl)imide anion is represented by the following formula (1): 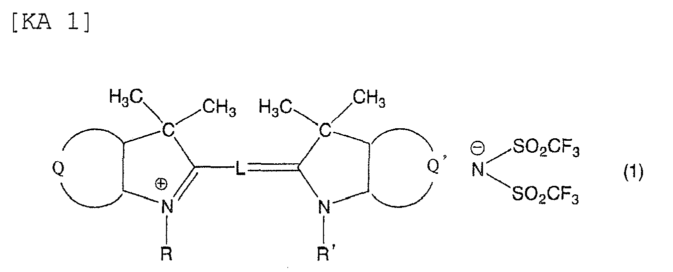 (wherein, in the above formula (1), each of Q and Q' independently represents a benzene ring or a naphthalene ring which may have a substituent; each of R and R' independently represents an alkyl group or an alkoxyalkyl group; and L represents a linking group to form a carbocyanine).

3. The optical filter according to claim 2, wherein L in the formula (1) is one group selected from the group consisting of carbocyanine crosslinking groups represented by the following formulae: 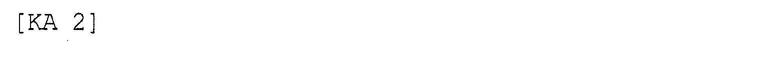 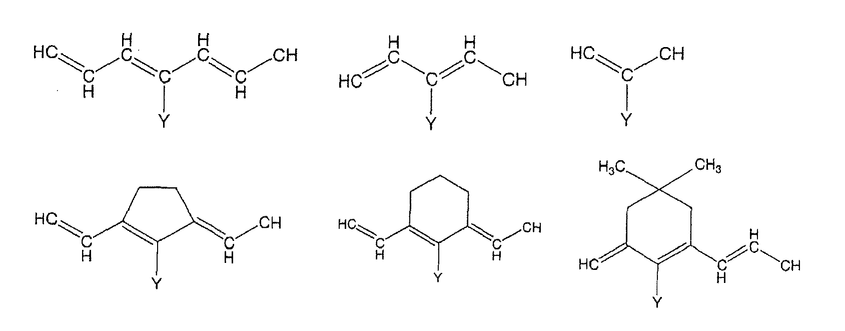 (wherein Y represents a halogen atom, a diphenylamino group, or a lower alkyl group).

4. The optical filter according to claim 2 or 3, wherein R and R' in the formula (1) are a methyl group or a methoxyethyl group.

5. The optical filter according to any one of claims 1 to 3, wherein said filter is for cutting-off near infrared rays, improving image characteristics, or both thereof.

6. The optical filter according to any one of claims 1 to 3, wherein said filter is for a plasma display panel.

7. The optical filter according to any one of claims 1 to 3, further comprising, as a near infrared rays absorbing agent, any coloring matter of a diimonium compound, a phthalocyanine type compound, or a nickel complex type compound.

8. The optical filter according to claim 7, wherein an anion of diimonium compound is the same anion as a cyanine coloring matter.

9. A cyanine compound represented by the following formula (1): 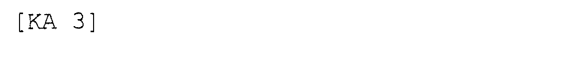 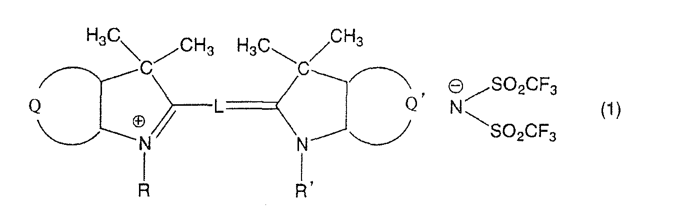 {wherein, in the above formula (1), each of Q and Q' independently represents a benzene ring which may be substituted by a halogen atom or methoxy, or a naphthalene ring which may be substituted by a halogen atom or methoxy; each of R and R' independently represents an alkyl group or an alkoxyalkyl group; and L represents any one of the linking groups represented by the following formulae: 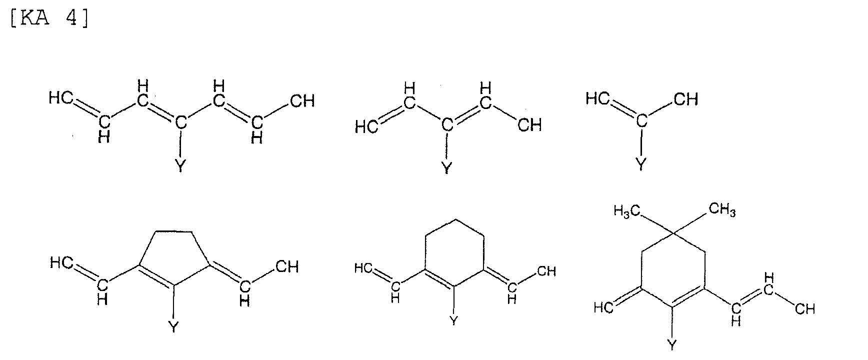 (wherein Y represents a halogen atom, a diphenylamino group, or a lower alkyl group)}.

10. The cyanine compound according to claim 9, wherein, in the formula (1), an indole group having Q or Q' linking to L is a group represented by an indole group represented by the following formula (4) (in the case of an indole group having Q' , the formula (4) should be read as a corresponding structural formula): 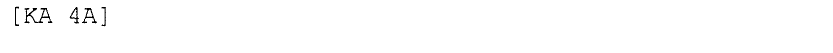 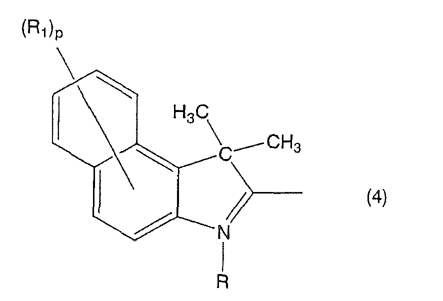 (wherein R₁ represents a halogen atom, an alkyl group, or an alkoxyalkyl group; p represents 0 or an integer not larger than 4; and R means the same as in the above formula (1)).

11. The cyanine compound according to claim 9 or 10, wherein, in the formula (1), the linking group represented by L is a linking group represented by the following formula (9): 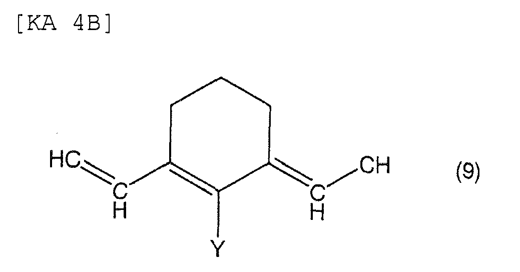 (wherein Y represents a halogen atom, a diphenylamino group, or a lower alkyl group).

12. The cyanine compound according to claim 9 or 10, wherein, in the formula (1), the linking group represented by L is a linking group represented by the following formula (8): 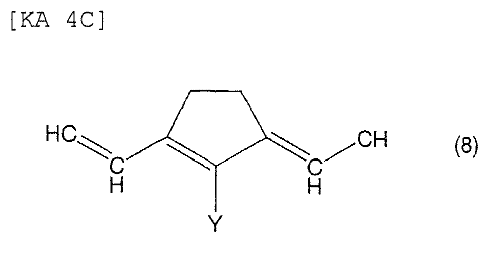 (wherein Y represents a halogen atom, a diphenylamino group, or a lower alkyl group).
